(19) Europäisches Patentamt
European Patent Office
Office européen des brevets

(11) Veröffentlichungsnummer : **0 066 210**
**B1**

(12) # EUROPÄISCHE PATENTSCHRIFT

(45) Veröffentlichungstag der Patentschrift : **11.04.84**

(51) Int. Cl.³ : **C 07 C 85/24**, C 07 C 87/40

(21) Anmeldenummer : **82104441.9**

(22) Anmeldetag : **21.05.82**

(54) **Katalytische Hydrierung von Di-(4-aminophenyl)methan.**

(30) Priorität : **01.06.81 US 269200**

(43) Veröffentlichungstag der Anmeldung : **08.12.82 Patentblatt 82/49**

(45) Bekanntmachung des Hinweises auf die Patenterteilung : **11.04.84 Patentblatt 84/15**

(84) Benannte Vertragsstaaten : **BE DE FR GB IT NL**

(56) Entgegenhaltungen :
**DE-A- 2 423 639**
**GB-A- 1 122 609**
**US-A- 2 606 925**
**US-A- 3 347 917**
**CHEMICAL ABSTRACTS, Band 77, Nr. 25, 18. Dezember 1972, Seite 368, Nr. 164129q, Columbus Ohio (USA)**

(73) Patentinhaber : **Mobay Chemical Corporation**
**Penn Lincoln Parkway West**
**Pittsburgh, Pennsylvania 15205 (US)**

(72) Erfinder : **Allen, Gary F.**
**811 Clearview Terrace**
**New Martinsville, WV 26155 (US)**

(74) Vertreter : **Drope, Rüdiger, Dr. et al**
**c/o Bayer AG Zentralbereich Patente Marken und Lizenzen**
**D-5090 Leverkusen 1, Bayerwerk (DE)**

**EP 0 066 210 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Katalytische Hydrierung von Di-(4-aminophenyl)methan

Bei der Herstellung von Di(4-aminocyclohexyl)methan durch katalytische Hydrierung von Di-(4-aminophenyl)-methan werden im wesentlichen drei Stereoisomere gebildet :

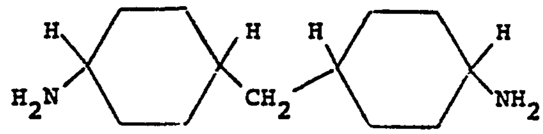

CIS CIS

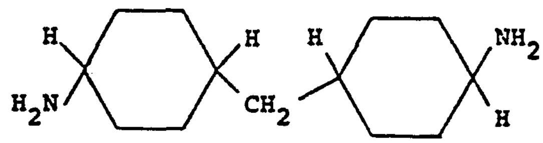

CIS TRANS

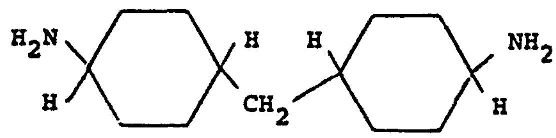

TRANS TRANS

Es ist in der Fachwelt bekannt, daß für die Herstellung eines entsprechenden Isocyanats (mittels des bekannten Verfahrens der Phosgenierung), das bei Raumtemperatur (d. h. von 20 °C bis 25 °C) flüssig und lagerbeständig ist, die Mischung der für die Phosgenierung eingesetzten Amin-Stereoisomeren das Trans, trans-Stereoisomere nur in relativ kleineren Mengen (typischerweise von 15 bis 40 Gew.-%) enthalten darf.

In der Fachwelt sind zahlreiche Verfahrensweisen zur Herstellung solcher Amin-Gemische bekannt, die die geforderte Menge des Trans, trans-Isomeren enthalten. Repräsentativ für diese Verfahrensweisen sind diejenigen, die in den US-PSen 3 153 088, 3 155 724, 3 393 236, 3 644 522, 3 711 550 und 3 766 272 beschrieben sind. Diese bekannten Methoden erfordern im allgemeinen die Abtrennung eines die geforderte Menge des Trans, trans-Isomeren enthaltenden Amin-Gemisches aus einem Amin-Gemisch, das durch die Hydrierung gebildet wird und etwa 50 Gew.-% des Trans, trans-Isomeren enthält. In der Fachwelt sind auch Verfahren zur direkten Herstellung eines die geforderte Menge des Trans, trans-Isomeren enthaltenden Amin-Gemischs aus Di(4-aminophenyl) methan bekannt, die die Zwischenstufe der Abtrennung entbehrlich machen (vgl. die US-PS 2 606 928). Jedoch sind hierbei die Reaktionsgeschwindigkeiten für Zwecke technischer Anwendungen viel zu niedrig.

Zahlreiche Verfahren zur Herstellung von Di-(4-amino-cyclohexyl)methan aus Di-(4-aminophenyl)-methan mittels katalytischer Hydrierung unter Verwendung von Ruthenium-Katalysatoren in Form von Trägerkatalysatoren oder trägerfreien Katalysatoren sind in der Fachwelt bekannt. Typische Verfahren dieser Art sind in den US-Psen 2 494 563, 2 606 924, 2 606 928, 2 606 925, 3 347 917, 3 676 495, 3 959 374, 3 743 677, 3 914 307, 3 825 586, 3 636 108 und 4 161 492 beschrieben. Wenngleich einige dieser Verfahren ein Amin-Gemisch liefern, das das Trans, trans-Isomere in einer Menge enthält, die die Herstellung flüssiger, lagerbeständiger Isocyanate ermöglicht, sind die Reaktionsgeschwindigkeiten viel zu niedrig für einen technischen Einsatz.

Es wurde auch beschrieben, daß Katalysatoren auf Ruthenium-Basis für die Hydrierung von

a) Polycycloaromatischen Polyaminen die aus Anilin und Formaldehyd aufgebaut sind (vgl. die US-PS 4 226 737),
b) 2,4-Bis(p-aminobenzyl)anilin (vgl. die US-PS 3 557 180),
c) 2,4'-Diaminodiphenylmethan (vgl. die US-PS 3 590 002),
d) Tolylendiamin/Formaldehyd-Kondensaten (vgl. die US-Psen 3 330 850 und 3 361 814) und
e) Di-(4-nitrophenyl)methan (vgl. die US-PS 3 742 049)

geeignet sind. Keines dieser Verfahren betrifft jedoch das hier vorliegende Problem, d. h. die Herstellung von Di-(4-aminocyclohexyl)methan, das das Trans, trans-Isomere in einer Menge von 15 bis 40 Gew.-% enthält.

Schließlich ist auch die Verwendung eines Lösungsmittels sowie von Ammoniak bei der Hydrierung

von Di-(4-aminophenyl)methan in Gegenwart eines Ruthenium-Katalysators bekannt (vgl. die US-PSen 3 347 917, 3 636 108 und 3 644 522). Die US-PS 3 347 917 beschreibt die Hydrierung von Di-(4-aminophenyl)methan bei Temperaturen von 180 °C bis 300 °C und Drücken oberhalb von 34,5 bar (500 psi) in Gegenwart von Ruthenium, Ammoniak und Lösungsmittel, wodurch in hoher Ausbeute in Di-(4-aminocyclohexyl)methan mit einem hohen Anteil des Trans, trans-Isomeren (d. h. oberhalb von 45 Gew.-%) erhalten wird. Die US-PS 3 636 108 beschreibt die Hydrierung von Di-(4-aminophenyl)methan bei Temperaturen von 100 °C bis 300 °C und Drücken oberhalb von 13,8 bar (200 psi) in Gegenwart eines Alkali-moderierten Ruthenium-Trägerkatalysators sowie gegebenenfalls von Ammoniak und Lösungsmittel. In denjenigen Beispielen dieser US-PS, für die der erhaltene Anteil des Trans, trans-Isomeren als niedrig angegeben wird (Beispiele 21, 22 und 27), wurden weder in organisches Lösungsmittel noch Ammoniak verwendet. Die US-PS 3 644 522 ist der vorgenannten ähnlich und unterscheidet sich dadurch von ihr, daß der Ruthenium-Trägerkatalysator ein spezielles Substrat als Träger enthält. Soweit der Trans, trans-Isomeren-Gehalt als niedrig beschrieben wurde (Beispiele 17, 18 und 19), wurden wie in der vorgennanten US-PS weder organisches Lösungsmittel noch Ammoniak verwendet.

Die vorliegende Erfindung betrifft ein Verfahren zur Herstelllung flüssigen, 15 bis 40 Gew.-% des Trans, trans-Isomeren enthaltenden Di-(4-aminocyclohexyl)methan aus di-(4-aminophenyl)methan durch Hydrierung in Gegenwart von (i) einem Ruthenium-auf-Aluminiumoxid-Katalysator (ii) einem aliphatischen Alkohol und (iii) Ammoniak und unter Einhaltung spezieller Verfahrensbedingungen. Im einzelnen sind während der Hydrierung ein Wasserstoff-Druck von mindestens 34,5 bar (500 psi) und eine Temperatur im Bereich von 150 °C bis 217 °C einzuhalten, und die Dauer der Hydrierung darf etwa 60 min nicht überschreiten.

Die aus Ruthenium auf Aluminiumoxid bestehenden Katalysatoren, die in der vorliegenden Erfindung verwendet werden, sind allgemein bekannt und im Handel erhältlich. Der zur Zeit bevorzugte Katalysator kann von der Firma Engelhard bezogen werden und enthält 5 Gew.-% Ruthenium auf einem Aluminiumoxid-Träger.

Bei der Durchführung des Verfahrens gemäß der vorliegenden Erfindung gelangen die allgemein in der Fachwelt angewandten Verfahrensweisen zum Einsatz, wobei die einzigen besonderen Anforderungen in der Anwesenheit des Alkohols und des Ammoniaks sowie den im Vorstehenden angegebenen Bedingungen für Druck und Temperatur bestehen.

Die Hydrierung muß in Gegenwart eines aliphatischen Alkohols durchgeführt werden. Die in der vorliegenden Erfindung verwendeten aliphatischen Alkohole umfassen sämtliche aliphatischen $C_1$- bis $C_{10}$-Alkohole. Speziell geeignete Alkohole sind unter anderen Methanol, Ethanol, n-Propanol und Isopropanol. Das zur Zeit bevorzugte Lösungsmittel ist Methanol. Die Menge des Alkohols muß mindestens 25 Gew.-%, bezogen auf das Gesamtgewicht von Alkohol und Ausgangs-Diamin, betragen. Die obere Grenze der Menge des Lösungsmittels wird durch wirtschaftliche Gesichtspunkte des Verfahrens bestimmt; im allgemeinen wird sie 60 Gew.-%, bezogen auf das Gesamtgewicht von Alkohol und Ausgangs-Diamin nicht überschreiten. Der bevorzugte Bereich der Alkohol-Menge beträgt 30 bis 50 Gew.-%, bezogen auf das Gesamtgewicht von Alkohol und Ausgangs-Diamin.

Ammoniak muß während der Hydrierungs-Reaktion ebenfalls anwesend sein. Im allgemeinen wird eine solche Menge Ammoniak eingesetzt, daß das Molverhältnis Ammoniak zu Ausgangs-Diamin von 0,5 : 1 bis 10 : 1 beträgt. Vorzugsweise wird eine Ammoniak-Menge entsprechend einen Molverhältnis von 0,5 : 1 bis 5 : 1 verwendet, wobei ein Wert um 1 : 1 besonders bevorzugt wird. Die höheren Ammoniak-Mengen sind allgemein weniger günstig, da hierbei im allgemeinen höhere Drücke erforderlich sind.

Es wird vorzugsweise eine solche Katalysator-Menge verwendet, daß die Menge des in der Reaktionsmischung vorhandenen Rutheniums mindestens 0,05 Gew.-%, bezogen auf das Gewicht des Ausgangs-Diamins, und vorzugsweise von 0,1 bis 3 Gew.-% beträgt, wobei ein Gehalt von 0,1 bis 1 Gew.-% besonders bevorzugt wird. Wirtschaftliche Überlegungen bestimmen im allgemeinen die einzusetzende Höchstmenge an Katalysator, da dieser im allgemeinen teuer ist.

Im allgemeinen werden die Stoffe miteinander vermischt und chargenweise dem Reaktionsgefäß zugeführt, jedoch kann auch ein kontinuierliches Verfahren angewandt werden.

Die Hydrierung wird durchgeführt bei einer Temperatur innerhalb des Bereichs von 150 °C bis 217 °C und vorzugsweise von 170 °C bis 200 °C. Die genaue Wahl der Temperatur in einem bestimmten Falle hängt von der gewünschten Reaktionsgeschwindigkeit und dem angestrebten Gehalt an dem Trans, trans-Isomeren ab. Im allgemeinen läuft die Reaktion umso schneller ab und ist der Trans, trans-Gehalt des Endprodukts umso höher, je höher die Temperatur ist. Die temperatur wird demnach im allgemeinen so gewählt, daß sie einen optimalen Kompromiß zwischen der Reaktionsdauer und dem Gehalt des Trans, trans-Isomeren ergibt.

Der bei dem Verfahren gemäß der vorliegenden Erfindung angewandte Wasserstoff-Druck muß mindestens bei 34,5 bar (500 psi) gehalten werden und liegt im allgemeinen zwischen 103,4 und 275,8 bar (1 500 bis 4 000 psi). Naturgemäß hängen die Drücke von den verwendeten Apparaturen ab. So können Drücke bis 551,6 bar (8 000 psi) oder höher verwendet werden, wenn geeignete Hochdruck-Apparaturen zur Verfügung stehen. Im allgemeinen wurde gefunden, daß die Ausbeute mit steigendem Druck zunimmt.

Das Fortschreiten der Reaktion wird in einfacher Weise durch Beobachtung der von der Reaktionsmischung aufgenommenen Wasserstoff-Menge verfolgt, und die Hydrierung wird zu dem Zeitpunkt

beendet, bei dem die theoretische Menge Wasserstoff absorbiert worden ist. Im allgemeinen übersteigt die Gesamtdauer der Hydrierung unter den angegebenen Bedingungen 60 min nicht; typischerweise beträgt die Hydrierzeit 10 bis 50 min. Längere Reaktionszeiten bewirken, insbesondere bei höheren Temperaturen, einen Anstieg des Gehalts des Trans, trans-Isomeren. Nach der Hydrierung wird der Katalysator von der Lösung des reduzierten Materials abgetrennt, und letztere wird destilliert, um das Di-(4-aminocyclohexyl)-methan daraus zu isolieren.

Die vorliegende Erfindung wird durch die folgenden Beispiele näher erläutert, ist jedoch nicht auf diese beschränkt. Hierin beziehen sich, sofern nicht anders vermerkt, sämtliche Angaben von Teilen und Prozentsätzen auf das Gewicht.

## Beispiele

### Beispiele 1 bis 14

200 Teile Di-(4-aminophenyl)methan, 200 Teile einer Methylalkohol-Lösung, die etwa 16 Teile Ammoniak enthielt, und die in Tabelle 1 angegebene Menge des Katalysators wurden in einen Hochdruck-Autoklaven gefüllt. Der verwendete Katalysator war ein Katalysator aus Ruthenium auf Aluminiumoxid von Engelhard mit einem Ruthenium-Gehalt von 5 %. Der Autoklav wurde verschlossen und auf den in Tabelle 1 angegebenen Druck gebracht, und der Inhalt wurde auf die in Tabelle 1 angegebene Temperatur erhitzt. Die Hydrierzeiten sind in Tabelle 1 aufgeführt. Der Inhalt wurde dem Autoklaven entnommen, unter Vakuum filtriert und dann destilliert, um den Methylalkohol und die niedrig siedenden Produkte auszutreiben. Die erhaltenen Hydrierungsprodukte (abgenommen als Kopfdestillat) wurden in bezug auf ihre Ausbeute und ihren Gehalt an dem Trans, trans-Isomeren analysiert. Die dabei erhaltenen Ergebnisse sind in Tabelle 1 aufgeführt.

Die Beispiele 6 und 11 bis 4 sind Vergleichbeispiele, die nicht in dem Rahmen der vorliegenden Erfindung fallen.

Tabelle 1

| Beispiel Nr. | Katalysator Menge Gew.-Tl. | Ru-Metall Gew.-% | Druck bar | (psi) | Temp. °C | Hydrier-Zeit min | Ausbeute mittels HPLC % | Gehalt Trans, trans-I. % |
|---|---|---|---|---|---|---|---|---|
| 1 | 15 | 0,5 | 137,9 | (2000) | 177 | 45 | 81 | 23 |
| 2 | 30 | 1 | 275,8 | (4000) | 177 | 20 | 100 | 38 |
| 3 | 30 | 1 | 275,8 | (4000) | 177 | 23 | 83 | 23 |
| 4 | 5 | 0,13 | 275,8 | (4000) | 180 | 45 | 85 | 26 |
| 5 | 5 | 0,13 | 275,8 | (4000) | 180 | 50 | 98 | 32 |
| 6*) | 30 | 1 | 103,4 | (1500) | 200 | 120 | 58 | 55 |
| 7 | 15 | 0,5 | 275,8 | (4000) | 200 | 10 | 89 | 26 |
| 8 | 15 | 0,5 | 103,4 | (1500) | 216 | 40 | 62 | 41 |
| 9 | 15 | 0,5 | 103,4 | (1500) | 216 | 44 | 91 | 40 |
| 10 | 15 | 0,5 | 275,8 | (4000) | 217 | 15 | 88 | 36 |
| 11*) | 5 | 0,13 | 275,8 | (4000) | 220 | 15 | 77 | 54 |
| 12*) | 30 | 1 | 275,8 | (4000) | 220 | 32 | 93 | 46 |
| 13*) | 15 | 0,5 | 69,0 | (1000) | 230 | 44 | 38 | 55 |
| 14*) | 30 | 1 | 137,9 | (2000) | 230 | 24 | 87 | 46 |

*) Vergleichsbeispiele außerhalb der beanspruchten Bedingungen.

**Ansprüche**

1. Verfahren zur katalytischen Hydrierung von Di-(4-aminophenyl)methan zu flüssigem, 15 bis 40 Gew.-% des Trans-trans-Isomeren enthaltenden Di-(4-aminocyclohexyl)methan, dadurch gekennzeichnet, daß Di-(4-aminophenyl)methan in Gegenwart von

(i) einem Katalysator von Ruthenium auf Aluminiumoxid,

(ii) mindestens 25 Gew.-% eines aliphatischen Alkohols, bezogen auf das Gesamtgewicht von Di-(4-aminophenyl)methan und dem Alkohol, und

(iii) Ammoniak in einer solchen Menge, daß das Molverhältnis Ammoniak zu Di-(4-aminophenyl)methan von 0,5 : 1 bis 10 : 1 beträgt,

unter einem Wasserstoff-Druck von mindestens 34,5 bar (500 psi) und bei einer Temperatur von 150 °C bis 217 °C während einer Zeitdauer von nicht mehr als 60 min hydriert wird.

2. Verfahren nach Anspruch 1, dadurch gekennzeichnet, daß der Alkohol 1 bis 10 Kohlenstoff-Atome enthält.

3. Verfahren nach Anspruch 1 und 2, dadurch gekennzeichnet, daß der Alkohol Methanol ist.

4. Verfahren nach Anspruch 1 bis 3, dadurch gekennzeichnet, daß die Menge Alkohol 25 bis 60 Gew.-% beträgt.

5. Verfahren nach Anspruch 1 bis 4, dadurch gekennzeichnet, daß das Molverhältnis von 0,5 : 1 bis 5 : 1 beträgt.

6. Verfahren nach Anspruch 1 bis 5, dadurch gekennzeichnet, daß eine solche Katalysator-Menge eingesetzt wird, daß dei Menge des Rutheniums mindestens 0,05 Gew.-% des Di-(4-aminophenyl)methans beträgt.

7. Verfahren nach Anspruch 1 bis 6, dadurch gekennzeichnet, daß die Menge des Rutheniums 0,1 bis 3 Gew.-% des Di-(4-aminophenyl)methans beträgt.

8. Verfahren nach Anspruch 1 bis 7, dadurch gekennzeichnet, daß die Temperatur 170 °C bis 200 °C beträgt.

9. Verfahren nach Anspruch 1 bis 8, dadurch gekennzeichnet, daß der Druck mindestens 103,4 bar (1 500 psi) beträgt.

10. Verfahren nach Anspruch 1 bis 9, dadurch gekennzeichnet, daß die Zeitdauer zwischen 10 und 50 min beträgt.

**Claims**

1. Process for the catalytic hydrogenation of di-(4-aminophenyl)methane to give liquid di-(4-aminocyclohexyl)methane containing 15 to 40 % by weight of the trans-trans isomer, characterised in that di-(4-aminophenyl)methane is hydrogenated in the presence of

(i) a catalyst of ruthenium on aluminium oxide,

(ii) at least 25 % by weight of an aliphatic alcohol, based on the total weight of di-(4-aminophenyl)methane and the alcohol, and

(iii) ammonia in such a quantity that the molar ratio of ammonia to di-(4-aminophenyl)methane is from 0.5 : 1 to 10 : 1,

under a hydrogen pressure of at least 34.5 bars (500 psi) and at a temperature of 150 °C to 217 °C for a time not exceeding 60 min.

2. Process according to Claim 1, characterised in that the alcohol contains 1 to 10 carbon atoms.

3. Process according to Claim 1 and 2, characterised in that the alcohol is methanol.

4. Process according to Claim 1 to 3, characterised in that the quantity of alcohol is 25 to 60 % by weight.

5. Process according to Claim 1 to 4, characterised in that the molar ratio is from 0.5 : 1 to 5 : 1.

6. Process according to Claim 1 to 5, characterised in that such an amount of catalyst is used that the quantity of ruthenium is at least 0.05 % by weight of the di-(4-aminophenyl)methane.

7. Process according to Claim 1 to 6, characterised in that the quantity of ruthenium is 0.1 to 3 % by weight of the di-(4-aminophenyl)methane.

8. Process according to Claim 7 to 7, characterised in that the temperature is 170 °C to 200 °C.

9. Process according to Claim 1 to 8, characterised in that the pressure is at least 103.4 bars (1,500 psi).

10. Process according to Claim 1 to 9, characterised in that the length of time is between 10 and 50 min.

**Revendications**

1. Procédé pour l'hydrogénation catalytique du di-(4-aminophényl)-méthane en di-(4-aminocyclohexyl)-méthane liquide contenant 15 à 40 % en poids de l'isomère trans, trans, caractérisé en ce que l'on hydrogène le di-(4-aminophényl)-méthane en présence de

(i) un catalyseur consistant en ruthénium sur alumine,

(ii) au moins 25 % en poids d'un alcool aliphatique par rapport au poids total du di-(4-aminophényl)-méthane et de l'alcool, et

(iii) de l'ammoniac en quantité correspondant à un rapport molaire ammoniac/di-(4-aminophényl)-méthane de 0,5 : 1 à 10 : 1,

sous une pression d'hydrogène d'au moins 34,5 bars (500 psi) et à une température de 150 à 217 °C pendant une durée ne dépassant pas 60 min.

2. Procédé selon la revendication 1, caractérisé en ce que l'alcool contient de 1 à 10 atomes de carbone.

3. Procédé selon les revendications 1 et 2, caractérisé en ce que l'alcool est le méthanol.

4. Procédé selon les revendications 1 à 3, caractérisé en ce que la quantité d'alcool est de 25 à 60 % en poids.

5. Procédé selon les revendications 1 à 4, caractérisé en ce que le rapport molaire est de 0,5 : 1 à 5 : 1.

6. Procédé selon les revendications 1 à 5, caractérisé en ce que l'on utilise le catalyseur en quantité telle que la quantité de ruthénium représente au moins 0,05 % du poids du di-(4-aminophényl)-méthane.

7. Procédé selon les revendications 1 à 6, caractérisé en ce que la quantité de ruthénium est de 0,1 à 3 % du poids du di-(4-aminophényl)-méthane.

8. Procédé selon les revendications 1 à 7, caractérisé en ce que la température est de 170 à 200 °C.

9. Procédé selon les revendications 1 à 8, caractérisé en ce que la pression est d'au moins 103,4 bars (1 500 psi).

10. Procédé selon les revendications 1 à 9, caractérisé en ce que la durée est de 10 à 50 min.